# EUROPÄISCHE PATENTSCHRIFT

(11) **EP 1 480 700 B1**
(45) Veröffentlichungstag und Bekanntmachung des Hinweises auf die Patenterteilung: **27.07.2005**
(21) Anmeldenummer: 03753432.8
(22) Anmeldetag: 19.09.2003
(51) Int. Cl.: A61M 5/32

(54) **VERFAHREN ZUR HERSTELLUNG EINER KANÜLE, INSBESONDERE FÜR DIE SPINALANÄSTHESIE**
METHOD FOR PRODUCING A CANNULA USED SPECIALLY FOR A SPINAL ANAESTHESIA
PROCEDE POUR REALISER UNE CANULE DESTINEE NOTAMMENT A LA RACHIANESTHESIE

(30) Priorität: 17.10.2002 DE 10248377
(43) Veröffentlichungstag der Anmeldung: 01.12.2004
(73) Patentinhaber: Haindl, Hans, Dr., 30974 Wennigsen (DE)
(72) Erfinder: Haindl, Hans, Dr., 30974 Wennigsen (DE)
(74) Vertreter: Leine, Sigurd, Dipl.-Ing.
(86) Internationale Anmeldenummer: PCT/EP2003/010443
(87) Internationale Veröffentlichungsnummer: WO 2004/035118

(56) Entgegenhaltungen:
- EP-A- 0 271 775
- DE-A- 19 717 253
- DE-U- 29 908 794
- US-A- 3 906 932

## Beschreibung

Die Erfindung betrifft ein Verfahren der im Oberbegriff des Anspruchs 1 genannten Art zur Herstellung einer Kanüle, insbesondere für die Spinalanästhesie.

Durch US 5 848 996 ist eine Kanüle bekannt, die für die Verwendung bei der Spinalanästhesie bestimmt ist. Sie weist ein starres Kanülenrohr auf, dessen Ende einen sich zu einer Einstechspitze hin ballig verjüngenden Bereich sowie zwei Austrittsöffnungen vor Beginn des sich verjüngenden Bereichs aufweist. Diese bekannte Kanüle ist als "Pencil-Point-Spinalkanüle" wegen ihrer bleistiftförmigen Spitze bezeichnet. Sie hat den Nachteil, daß der Abstand der Austrittsöffnung von der Spitze verhältnismäßig groß ist. Dies macht es bei der Spinalanästhesie erforderlich, so tief in den Spinalraum einzustechen, bis die Austrittsöffnungen mit dem Spinalraum in Verbindung stehen. Wegen des so erforderlichen weiten Einstichs können sich Verletzungen der Nervenfasern ergeben. Da die Austrittsöffnungen rund sind, kann ihr Querschnitt nur gering sein, so daß sich bei Austritt einer Flüssigkeit aus den Austrittsöffnungen eine verhältnismäßig große Austrittsgeschwindigkeit ergibt. Außerdem ist die Herstellung dieser bekannten Kanüle kompliziert und aufwendig.

Durch DE 199 11 970 A1 ist eine Kanüle der zuvor genannten Art bekannt. Die Spitze ist im wesentlichen kegelförmig und geschlossen ausgebildet, und die zumindest eine Fluiddurchtrittsöffnung ist von einem Längsschlitz in der seitlichen Wandung des Schaftes gebildet. Die Herstellung der kegelförmigen Spitze und der schmalen Schlitze ist kompliziert. Außerdem wirken die Schlitze als Düse, so daß eine austretende Flüssigkeit eine verhältnismäßig hohe Geschwindigkeit hat, was nicht erwünscht ist. Ein Verfahren zur Herstellung dieser bekannten Kanüle ist nicht angegeben. Offensichtlich wird zunächst die kegelförmige Spitze hergestellt, wonach in einem gesonderten Arbeitsgang die schmalen Schlitze hergestellt werden.

Durch US 3 906 932 ist eine Kanülenspitze zum Durchstechen eines Pfropfens zum Verschluß eines Behälters bekannt, der während des Eindringens in den Pfropfen nicht zu einer Seite ausgelenkt wird. Zu diesem Zweck ist das Ende eines zylindrischen Rohres auf zwei gegenüberliegenden Seiten schräg angeschliffen und die so entstandenen Spitzen sind aufeinanderzu gebogen und bilden so eine Schneide, die das Durchstechen des Pfropfens erleichtert. Eine derartige Kanüle ist zur Vewendung in der Medizin nicht geeignet, da sie das durchstochene Gewebe nicht aufweiten, sondern durchschneiden würde. Das durch diese Schrift bekannte Verfahren ist somit nicht zur Herstellung einer in der Medizin verwendbaren Kanüle geeignet.

Der Erfindung liegt die Aufgabe zugrunde, ein Verfahren zur Herstellung einer Kanüle der im Oberbegriff des Anspruchs 1 genannten Art zu schaffen, das einfach durchzuführen ist und zu einer Kanüle führt, deren Austrittsöffnung einen vergrößerten Querschnitt hat und sich insbesondere so weit wie möglich nahe der Einstechspitze befindet.

Diese Aufgabe wird durch die im Kennzeichen des Anspruchs 1 angegebene Lehre gelöst.

Der diesem Verfahren zugrundeliegende Gedanke besteht darin, die Austrittsöffnung nicht wie bei den bekannten Kanülen nach der Verformung des Endes des Kanülenrohres zu einer balligen Spitze zu schaffen, vielmehr das Kanülenrohr in seinem Endbereich vor dessen Verformung so auszubilden, daß bei der Verformung zwangsläufig eine Austrittsöffnung entsteht. Erfindungsgemäß wird zur Bildung des kegeligen Bereichs ausgehend von einem bis zu seinem Ende zylindrischen Kanülenrohr das distale Ende des Kanülenrohres vorzugsweise bis zu einem äußersten distalen Ende hin abgeschrägt. Diese Abschrägung kann natürlich jede Form haben, sie kann beispielsweise durch einen geraden Schliff oder auch durch einen konvexen oder konkaven Schliff erzeugt werden. Die im abgeschrägten Bereich entstandenen Ränder werden entsprechend einer im wesentlichen kegeligen Umhüllenden so weit zusammengebogen, daß sie sich am äußersten distalen Rand des abgeschrägten Bereichs berühren und so eine Spitze bilden, im Bereich dahinter jedoch einen Abstand voneinander haben und so die Austrittsöffnung bilden. Durch das Zusammenbiegen der durch die Abschrägung entstandenen Ränder zur Bildung einer kegeligen Form entsteht somit die Austrittsöffnung automatisch in einem einzigen Arbeitsgang.

Eine zweckmäßige Weiterbildung des Grundgedankens des erfindungsgemäßen Verfahrens besteht darin, daß das distale Ende des Kanülenrohres auf zwei sich diametral gegenüberliegenden Seiten abgeschrägt und die jeweils in den abgeschrägten Bereichen entstandenen, paarweise gegenüberliegenden Ränder entsprechend einer kegeligen Umhüllenden so weit zusammengebogen werden, daß sie sich am äußersten distalen Rand berühren und eine Spitze bilden, im Bereich dahinter jedoch einen Abstand voneinander haben und so zwei Austrittsöffnungen bilden. Auf diese Weise ist der gesamte Austrittsquerschnitt verdoppelt.

Gemäß einer Weiterbildung dieses Verfahrens erfolgt das Zusammenbiegen der Ränder im äußersten distalen Bereich zur kegeligen Verengung des Querschnitts so weit, daß zwischen Einstechspitze und Austrittsöffnung eine geschlossene Umfangsfläche gebildet ist. Hierdurch werden die Einstecheigenschaften verbessert.

Vorteilhafterweise werden die Ränder der Wandung des Kanülenrohres im abgeschrägten Bereich vor dem Zusammenbiegen abgerundet. Dadurch ist auch nach der Verformung der Rand der Austrittsöffnung abgerundet. Die Durchführung der Abrundung ist jedem Fachmann bekannt.

Gemäß einer Ausführungsform des erfindungsgemäßen Verfahrens wird das Kanülenrohr während des Zusammenbiegens radial geführt. Dabei wird die Verformung im sich verjüngenden Bereich symmetrisch zu der in diesen Bereich verlängert gedachten Mittellinie des Kanülenrohres durchgeführt, derart, daß bei Abschluß der Verformung die erzeugte Einstechspitze auf dieser Mittellinie liegt.

Zur Durchführung dieser Ausführungsform des Verfahrens erfolgt die Umformung, d.h. das Zusammenbiegen der Ränder im abgeschrägten Bereich zweckmäßigerweise mittels zweier im Gleichtakt aufeinanderzu bewegter Formbacken, wobei das Kanülenrohr während der Verformung relativ zu den Formbacken gedreht wird. Auf diese Weise entsteht eine axialsymmetrische Form im sich verjüngenden Bereich. ,

Anhand einer Zeichnung soll die Erfindung an einem Ausführungsbeispiel näher erläutert werden.
- Fig. 1: zeigt den Endbereich einer Ausführungsform einer nach dem erfindungsgemäßen Verfahren hergestellten Kanüle mit Blick auf die Austrittsöffnung,
- Fig. 2: ist eine Seitenansicht von links in Fig. 1,
- Fig. 3: ist eine Ansicht Schnitt III-III in Fig. 2 und
- Fig. 4: ist ein Schnitt IV-IV durch Fig. 2.

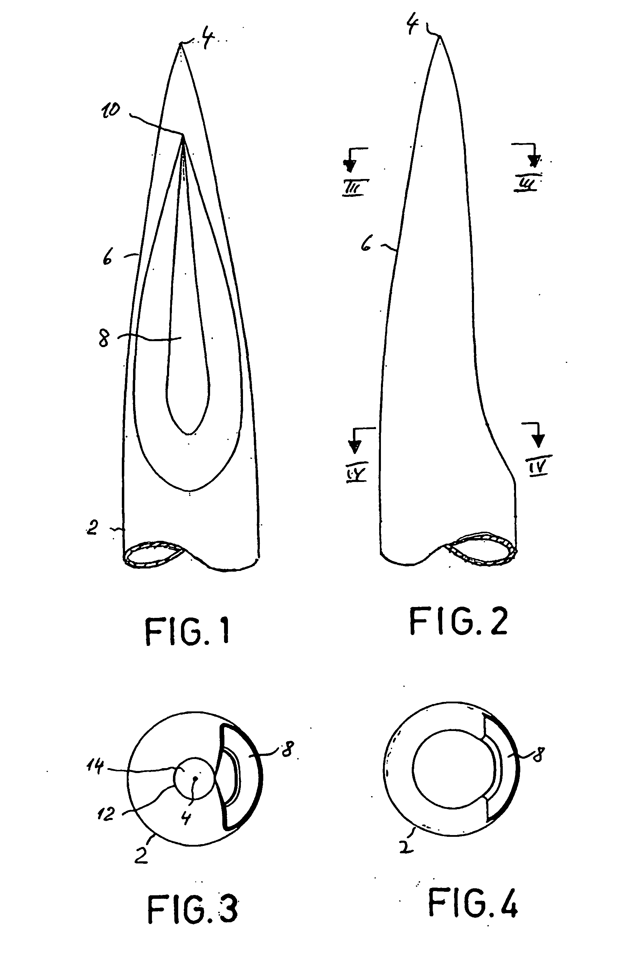

Fig. 1 zeigt das abgeschnittene distale Ende eines Kanülenrohres 2, das einen sich zu einer Einstechspitze 4 hin verjüngenden Bereich 6 aufweist, in dem sich eine tropfenförmige Austrittsöffnung 8 befindet. Deren Spitze 10 ist der Einstechspitze 4 zugewandt. Sie liegt entfernt von der Einstichspitze 4, so daß die Austrittsöffnung 8 entfernt von der Einstichspitze 4 endet. Auf diese Weise ist zwischen der Einstichspitze 4 und der Spitze 10 der Austrittsöffnung 8 eine geschlossene Umfangsfläche gebildet, die ein Einstechen in ein Gewebe erleichtert.

Fig. 2 zeigt eine Seitenansicht von links in Fig. 1.

Fig. 3 zeigt eine Ansicht III-III in Fig. 2. Es ist erkennbar, daß von der Spitze 4 bis zu einer durch eine Ringlinie 12 gekennzeichnete geschlossene Umfangsfläche 14 erstreckt.

Fig. 4 zeigt einen Schnitt IV-IV durch Fig. 2. Es ist erkennbar, daß die Austrittsöffnung 8 einen großen Querschnitt hat, so daß bei Verwendung der Kanüle eine große Menge Flüssigkeit mit geringer Geschwindigkeit austreten kann.

Das erfindungsgemäße Verfahren wird anhand der Fig. 1 und 2 nachfolgend näher erläutert. Zunächst wird das Kanülenrohr 2, das sich im Ausgangszustand zylindrisch bis zu der späteren Einstechspitze 4 hin erstreckt, bis zu dieser über den gesamten Querschnitt abgeschrägt. Dieses abgeschrägte Ende wird, indem das Kanülenrohr 2 radial geführt ist, zwischen zwei im Gleichtakt aufeinanderzu bewegter Formbacken geführt, die symmetrisch zu einer Mittellinie angeordnet sind, die durch die verlängert gedachte Mittellinie des Kanülenrohres 2 gebildet ist. Während sich die Formbacken im Gleichtakt aufeinanderzu und somit gegen das abgeschrägte Ende des Kanülenrohres 2 bewegen, wird dieses gedreht. Dadurch werden die durch die Abschrägung entstandenen Ränder entsprechend einer kegeligen Umhüllenden zusammengebogen. Dieser Verformungsvorgang wird so lange fortgesetzt, bis im Spitzenbereich die geschlossene Umfangsfläche 14 gebildet ist und sich die Ränder des abgeschrägten Kanülenrohres so aufeinanderzu bewegt haben, daß zwischen ihnen die Austrittsöffnung 8 gebildet ist. Vor Beginn des Verformungsvorganges werden die Ränder des abgeschrägten Endes des Kanülenrohres 2 abgerundet, so daß damit auch die Ränder der Austrittsöffnung 8 nach dem Verformungsvorgang abgerundet sind, so daß bei dem Einstechen der Kanüle in ein Gewebe kein Ausstechen von Gewebeteilen erfolgt.

## Patentansprüche

1. Verfahren zur Herstellung einer Kanüle, insbesondere für die Spinalanästhesie, die ein starres Kanülenrohr (2) aufweist, dessen distales Ende einen sich zu einer Einstechspitze (4) kegelig verjüngenden Bereich (6) und eine seitliche Austrittsöffnung (8) darin aufweist, **dadurch gekennzeichnet, daß** zur Bildung des kegeligen Bereichs (6) ausgehend von einem bis zu seinem Ende zylindrischen Kanülenrohr (2) zunächst das distale Ende des Kanülenrohres (2) vorzugsweise bis zu seinem äußersten distalen Ende hin nur auf einer Seite abgeschrägt und die so im abgeschrägten Bereich entstandenen Ränder entsprechend einer im wesentlichen kegeligen Umhüllenden so weit zusammengebogen werden, daß sie sich am äußersten distalen Rand des abgeschrägten Bereichs berühren und so eine Spitze bilden, im Bereich dahinter jedoch einen Abstand voneinander haben und so die Austrittsöffnung (8) bilden.

2. Verfahren nach Anspruch 1, **dadurch gekennzeichnet, daß** das Zusammenbiegen der Ränder im äußersten distalen Bereich so weit erfolgt, daß zwischen Einstechspitze (4) und Austrittsöffnung (8) eine geschlossene Umfangsfläche gebildet ist.

3. Verfahren nach Anspruch 1, **dadurch gekennzeichnet, daß** die Ränder der Wandung des Kanülenrohres (2) im abgeschrägten Bereich vor dem Zusammenbiegen abgerundet werden.

4. Verfahren nach Anspruch 1, **dadurch gekennzeichnet, daß** das Kanülenrohr (2) während des Zusammenbiegens radial geführt wird.

5. Verfahren nach Anspruch 4, **dadurch gekennzeichnet, daß** das Zusammenbiegen symmetrisch zu der in den sich verjüngenden Bereich (6) verlängert gedachten Mittellinie des Kanülenrohrs (2) durchgeführt wird, derart, daß bei Abschluß des Zusammenbiegens die erzeugte Einstechspitze (4) auf dieser Mittellinie liegt.

6. Verfahren nach Anspruch 5, **dadurch gekennzeichnet, daß** das Zusammenbiegen mittels zweier im Gleichtakt aufeinanderzu bewegter Formbacken erfolgt und daß das Kanülenrohr (2) während des Zusammenbiegens relativ zu den Formbacken gedreht wird.

## Claims

1. A method for the manufacture of a cannula, in particular for spinal anaesthesia, which comprises a rigid cannula tube (2), the distal end of which comprises a region (6) tapering conically to an insertion point (4) and a lateral outlet aperture (8) therein,
**characterised in that** to form the conical region (6) starting from a cannula tube (2) that is cylindrical right to its end, firstly the distal end of the cannula tube (2) is preferably chamfered on just one side right to its outermost distal end and the edges produced in this manner in the chamfered region are bent together corresponding to a substantially conical envelope so that they touch each other at the outermost distal edge of the chamfered region and thus form a point, but in the region therebehind are spaced from each other and thus form the outlet aperture (8).

2. A method according to Claim 1,
**characterised in that** the bending together operation of the edges in the outermost distal region takes place to the extent that a closed circumferential surface is formed between the insertion point (4) and the outlet aperture (8).

3. A method according to Claim 1,
**characterised in that** the edges of the wall of the cannula tube (2) are rounded in the chamfered region prior to the bending together operation.

4. A method according to Claim 1,
**characterised in that** the cannula tube (2) is radially guided during the bending together operation.

5. A method according to Claim 4,
**characterised in that** the bending together operation is performed symmetrically to the imaginary centre line of the cannula tube (2) extending in the tapering region (6) in such a manner that the produced insertion point (4) lies on this middle line at the conclusion of the bending together operation.

6. A method according to Claim 5,
**characterised in that** the bending together operation takes place by means of two shaping jaws moved towards each other in synchronism
and **in that** the cannula tube (2) is rotated relative to the shaping jaws during the bending together operation.

## Revendications

1. Procédé de fabrication d'une canule, en particulier pour l'anesthésie spinale, qui comporte un tube de canule (2) rigide dont l'extrémité distale présente une zone (6) se rétrécissant de manière conique en une pointe à piquer (4) et un orifice de sortie (8) latéral, **caractérisé en ce que** pour former la zone conique (6), en partant d'un tube de canule (2) cylindrique jusqu'à son extrémité, on ne chanfreine d'abord que sur un coté de l'extrémité distale du tube de canule (2) de préférence jusqu'à son extrémité distale la plus extérieure, et les bords ainsi formés dans la zone chanfreinée sont repliés l'un vers l'autre suivant une enveloppante sensiblement conique, suffisamment pour qu'ils se touchent sur le bord distal le plus extérieur de la zone chanfreinée, et forment ainsi une pointe, mais sont écartés l'un de l'autre dans la zone située à l'arrière et forment ainsi l'orifice de sortie (8).

2. Procédé selon la revendication 1, **caractérisé en ce qu'**on replie l'un vers l'autre les bords dans la zone distale la plus extérieure suffisamment pour qu'il se forme une surface périphérique fermée entre la pointe à piquer (4) et l'orifice de sortie (8).

3. Procédé selon la revendication 1, **caractérisé en ce que** les bords de la paroi du tube de canule (2) dans la zone chanfreinée sont arrondis avant d'être repliés l'un vers l'autre.

4. Procédé selon la revendication 1, **caractérisé en ce que** le tube de canule (2) est guidé radialement pendant le repliage.

5. Procédé selon la revendication 4, **caractérisé en ce que** le repliage est exécuté symétriquement par rapport à la ligne médiane imaginaire du tube de canule (2), prolongée dans la zone (6) se rétrécissant, de manière qu'à l'achèvement de l'opération de repliage la pointe à piquer (4) produite se situe sur cette ligne médiane.

6. Procédé selon la revendication 5, **caractérisé en ce que** le repliage s'effectue au moyen de deux mâchoires de formage déplacées de manière synchrone l'une vers l'autre, et **en ce que** le tube de canule (2) est tourné par rapport aux mâchoires de formage, pendant le repliage.
